## Europäisches Patentamt
## European Patent Office
## Office européen des brevets

(19)

(11) Veröffentlichungsnummer: **0 054 855**
**B1**

(12) # EUROPÄISCHE PATENTSCHRIFT

(45) Veröffentlichungstag der Patentschrift:
**13.02.85**

(21) Anmeldenummer: **81110329.0**

(22) Anmeldetag: **11.12.81**

(51) Int. Cl.⁴: **C 07 C 5/27**, C 07 C 11/09,
B 01 J 27/06, B 01 J 27/32

(54) **Verfahren zur Herstellung von i-Alkenen, hierzu geeigneter Katalysator und Verfahren zu dessen Herstellung.**

(30) Priorität: **23.12.80 DE 3048693**
**29.04.81 DE 3117053**
**19.09.81 DE 3137383**

(43) Veröffentlichungstag der Anmeldung:
**30.06.82 Patentblatt 82/26**

(45) Bekanntmachung des Hinweises auf die Patenterteilung:
**13.02.85 Patentblatt 85/7**

(84) Benannte Vertragsstaaten:
**DE GB NL**

(56) Entgegenhaltungen:
**EP - A - 0 032 543**
**DE - A - 1 518 580**
**DE - B - 1 518 584**
**DE - B - 1 518 595**
**DE - C - 869 061**
**US - A - 3 919 340**
**US - A - 4 014 948**

(73) Patentinhaber: **EC ERDÖLCHEMIE GMBH,**
**Postfach 75 20 02, D-5000 Köln 71 (DE)**

(72) Erfinder: **Müller, Hans Joachim, Dr., Heidberg 10a,**
**D-5090 Leverkusen-Niederblecher (DE)**
Erfinder: **Hönig, Helmar, Dr., Flottbektal 2,**
**D-2000 Hamburg 52 (DE)**
Erfinder: **Horlitz, Werner, Hackhauser Strasse 32,**
**D-4047 Dormagen 1 (DE)**

(74) Vertreter: **Mann, Volker, Dr. et al, c/o Bayer**
**Aktiengesellschaft Zentralbereich Patente Marken und**
**Lizenzen, D-5090 Leverkusen-Bayerwerk (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Die Erfindung betrifft ein Verfahren zur Herstellung von i-Alkenen durch katalytische Umwandlung von n-Alkenen, einen Katalysator, der für diese Umwandlung geeignet ist, und ein Verfahren zur Herstellung eines solchen Katalysators.

Es ist allgemein bekannt, daß sich n-Paraffine mit beispielsweise 4 bis 7 C-Atomen unter Verwendung geeigneter saurer Katalysatoren im Temperaturbereich von 100 bis 250° C zu den entsprechenden isomeren Paraffinen umwandeln lassen. Beispiele dafür sind zahlreiche in der Petrochemie und Mineralölindustrie angewandte Isomerisierungsprozesse zur Erhöhung der Oktanzahl leichter paraffinischer Erdölfraktionen. Weiterhin ist bekannt, daß sich im Gegensatz hierzu Olefine gleicher Kohlenstoffzahl nicht bzw. nur unter erschwerten Bedingungen, beispielsweise bei sehr hohen Temperaturen und mit schlechter Ausbeute, zu den entsprechenden Isoolefinen umwandeln lassen. Die bisher in der Literatur beschriebenen Versuche zur direkten Skelettisomerisierung von beispielsweise n-Buten zu i-Buten bzw. von n-Penten zu i-Penten an fest angeordneten Katalysatoren sind gekennzeichnet durch nur anfangs hohe Umsätze und Selektivitäten, die nach kurzer Bertriebszeit, oft schon nach wenigen Stunden abklingen und sich wesentlich verschlechtern. Die Verschlechterung der Ausbeuten und Selektivitäten ist im allgemeinen auf den Verlust an aktiv wirksamer Katalysatoroberfläche oder auf den Verlust an aktiven Zentren zurückzuführen. Dadurch werden hohe Verkokungsraten, Oligomerenbildung und Crackreaktionen beobachtet.

So wird in US 3 531 542 ein Verfahren zur Gewinnung von i-Buten aus n-Buten beschrieben, bei dem mehrstufig ein im Festbett angeordneter $Al_2O_3$-Katalysator eingesetzt wird. In US 3 663 453 wird die gleiche Umwandlung, ebenfalls in einem Festbett, mit einem Katalysator, bestehend aus Zirkoniumoxid und einem $Al_2O_3/ZrOCl$-Katalysator, durchgeführt. In US 2 568 964 wird ebenfalls die katalytische Isomerisierung von olefinischen Kohlenwasserstoffen in einem Festbett beschrieben. Es wird berichtet, daß beim Isomerisierungsprozeß Kohlenstoffablagerungen auf dem Katalysatormaterial eintreten, die die Aktivität reduzieren und ein periodisches Regenerieren des Katalysators notwendig machen. Nach der Regeneration soll der Katalysator seine volle Aktivität wieder erhalten, jedoch bleibt zumindest der Nachteil, daß während der Regenerierungsperiode der eigentliche Isomerisierungsprozeß unterbrochen werden muß.

Stellvertretend für andere Alkene sei am Beispiel der Butene folgender Stoffverbund genannt: In petrochemischen Anlagen oder in Raffinerien fallen vielfach Destillatfraktionen an, die n-Butene, gegebenenfalls im Gemisch mit i-Buten, i-Butan und n-Butan enthalten, so beispielsweise nach der Abtrennung von 1,3-Butadien aus einem $C_4$-Schnitt oder bei der Crackung von Wachsdestillaten. Das gegebenenfalls vorhandene i-Buten in solchen Destillatfraktionen wird vor allem in katalytischen Reaktionen umgesetzt, beispielsweise mit Methanol zu Methyl-tert.-butylether, welcher vom restlichen $C_4$-Schnitt destillativ abgetrennt und als Klopfzahlverbesserer für Fahrbenzin bzw. als Lösungsmittel verwendet werden kann. Eine weitere katalytische Umsetzung von i-Buten in solchen Gemischen ist die Oligomerisierung an sauren Katalysatoren zu beispielsweise $C_8$-, $C_{12}$- oder $C_{16}$-Oligomeren des Isobutens, die ebenfalls destillativ abgetrennt werden können. In beiden Fällen verbleibt als Restprodukt ein Gemisch von n-Butenen, n-Butan und i-Butan, welches in den meisten Fällen nur einem untergeordneten Verwendungszweck, beispielsweise dem Heizgas, zugeführt werden kann.

Es besteht daher der Wunsch, aus dem aus den obengenannten Reaktionen verbleibenden n-Buten/n-Butan/i-Butan-Gemisch als Restprodukt durch eine katalytische Umwandlung erneut ein i-Buten-haltiges Gemisch herzustellen, welches wiederum Reaktionen zugeführt werden kann, die beispielhaft oben beschrieben sind.

Es wurde ein Verfahren zur Herstellung von i-Alkenen durch katalytische Umwandlung von n-Alkenen im Gemisch mit Wasserstoff und gegebenenfalls mit Inertgasen bei erhöhter Temperatur an einem mit Halogenverbindungen von Elementen der dritten bis fünften Hauptgruppe und zweiten bis achten Nebengruppe des Periodensystems (Mendelejew) und/oder Halogenwasserstoffsäuren dotierten $Al_2O_3$- und/oder $SiO_2$-Katalysator gefunden, daß dadurch gekennzeichnet ist, daß ein speziell aktiviertes $Al_2O_3$ und/oder $SiO_2$ eingesetzt wird, wobei die spezielle Aktivierung darin besteht, daß ein Aluminiumhydroxid und/oder Aluminiumoxidhydrat und/oder Kieselsäuregel mit einem Gehalt an flüchtigen Ammoniumsalzen vor der Dotierung unter Austreibung der flüchtigen Ammoniumsalze zu $Al_2O_3$ und/oder $SiO_2$ kalziniert wird und daß in einem bewegten Katalysatorbett gearbeitet wird und gegebenenfalls der Katalysator ohne Verfahrensunterbrechung ganz oder teilweise durch neuen und/oder regenerierten Katalysator ersetzt wird.

Das im erfindungsgemäßen Verfahren einzusetzende Gemisch enthält ein n-Alken, beispielsweise mit 4—8, bevorzugt 4—5, besonders bevorzugt 4 C-Atomen und endständiger oder innenständiger Doppelbindung. Im Falle des Butens kann es also sowohl n-Buten-1, n-Buten-2 oder ein Gemisch absolut beliebiger Zusammensetzung dieser beiden sein. Andere erfindungsgemäß einsetzbare n-Alkene sind beispielsweise: n-Penten-1, n-Penten-2, n-Hexen-1, n-Hexen-2, n-Hexen-3, n-Hepten-1, -2 oder -3 sowie n-Octen-1, -2, -3 oder -4. Diese n-Alkene oder eines von ihnen können jedoch auch von anderen Kohlenwasserstoffen begleitet sein. Zu diesen Kohlenwasserstoffen zählen in erster Linie solche mit der gleichen Anzahl von C-Atomen, im Falle der Butene beispielsweise n-Butan und i-Butan. Ebenso können die erfindungsgemäß einsetzbaren n-Alkene neben den genannten Stoffen bereits

2

geringe Mengen i-Alken enthalten. Solche Gemische ergeben sich aus dem in einer petrochemischen Anlage gewünschten Material- und Verfahrensverbund beispielsweise nach der Entfernung von anderen Wertstoffen, wie Aromaten, i-Alkenen oder Diolefinen, wie er anhand der $C_4$-Kohlenwasserstoffe durch das folgende Beispiel illustriert werden soll:

Nach der Abtrennung des Butadiens aus einem aus der thermischen Crackung nach einem der bekannten Verfahren gewonnenen $C_4$-Schnitt verbleibt ein i-Buten-haltiges $C_4$-Gemisch folgender Zusammensetzung: 35 bis 45 Gew.-% i-Buten, 30 bis 50 Gew.-% n-Butene, 8 bis 15 Gew.-% n-Butan und 4 bis 6 Gew.-% i-Butan. Nach der Entfernung des Isobutens, beispielsweise durch katalytische Umsetzung mit Methanol zu Methyl-tert.-butylether oder durch Umsetzung des Isobutens zu seinen Oligomeren, verbleibt ein Rest-$C_4$-Kohlenwasserstoffgemisch mit etwa folgender Zusammensetzung: 0 bis 2 Gew.-% i-Buten, 60 bis 80 Gew.-% n-Butene, 10 bis 30 Gew.-% n-Butan und 5 bis 10 Gew.-% i-Butan. Dieses verbleibende Rest-$C_4$-Gemisch kann erfindungsgemäß eingesetzt werden.

Auch geringe Gehalte, beispielsweise bis zu je 10 Gew.-%, von nicht sauber abgetrennten Anteilen der benachbarten $C_3$-Kohlenwasserstoff-Fraktion und der $C_5$-Kohlenwasserstoff-Fraktion im Falle der n-Butene oder der anderen jeweiligen Nachbarfraktionen im Falle von Alkenen mit mehr als 4 C-Atomen sind für das erfindungsgemäße Verfahren unkritisch.

Das im erfindungsgemäßen Verfahren einzusetzende Gemisch ist weiterhin durch einen Gehalt an Wasserstoff gekennzeichnet. Dieser Wasserstoff wird im allgemeinen dem oder den n-Alkenen oder einem diese enthaltenden Gemisch vor Eintritt in den mit Katalysator gefüllten Reaktor zugesetzt. Hierzu sei beispielsweise eine Menge von 0,01 bis 15 Vol.-Teile, bevorzugt 0,1 bis 10 Vol.-Teile, besonders bevorzugt 0,5 bis 5 Vol.-Teile Wasserstoff pro 1 Vol.-Teil Gesamtmenge an Kohlenwasserstoffen im Einsatzprodukt genannt. Für den Fall, daß das einzusetzende Kohlenwasserstoffgemisch, das n-Alkene enthält, aus einem vorangeschalteten Raffinerieprozeß bereits Wasserstoff in der genannten Größenordnung enthält, kann selbstverständlich der Zusatz von weiterem Wasserstoff entfallen.

Als Wasserstoff kann reiner oder technischer Wasserstoff zugesetzt werden. Technischer Wasserstoff kann beispielsweise geringe Menge Kohlenwasserstoff, wie Methan oder Ethan, oder Inertgase, wie Stickstoff, Kohlendioxid oder Edelgase enthalten. Der Gehalt solcher Stoffe im technischen Wasserstoff wird auf die Einsatzmenge Wasserstoff angerechnet, ist aber ansonsten völlig unkritisch. Als Gehalt solcher Stoffe im technischen Wasserstoff sei beispielsweise 0,1 bis 20 Vol.-% genannt. Auch der Wasserstoff, der aus dem im erfindungsgemäßen Verfahren anfallenden Gemisch abgetrennt wird, kann wieder als Zumischung zum Eingangsprodukt verwendet werden.

Dem erfindungsgemäß einzusetzenden Gemisch, das mindestens ein n-Alken und Wasserstoff enthält, können weiterhin Inertgase zugesetzt werden, wie Wasserdampf, Stickstoff, Kohlendioxid oder Argon. Solche Inertstoffe sind bereits teilweise als mögliche Begleiter von technischem Wasserstoff genannt. Bevorzugt unter diesen Inertstoffen ist der Zusatz von Wasserdampf. Solche Inertstoffe können in einer großen Variationsbreite zugesetzt werden, beispielsweise 1 bis 50 Vol.-%, bevorzugt 5 bis 30 Vol.-%, bezogen auf das Gesamtgemisch aus n-Alkenen, Wasserstoff und den genannten Kohlenwasserstoffen als Begleitverbindungen.

Zur Aktivitätsverbesserung des Kontaktes können halogenierte Kohlenwasserstoffe, wie z. B. Tetrachlorkohlenstoff, Chloroform, chlorierte Ethane, Dichlorpropan, Fluorchlorethan und ähnliche dem Reaktoreinsatzprodukt zugesetzt werden. Es werden Mengen von diskontinuierlich 0,1—1% und kontinuierlich 1—100 ppm, bezogen auf das gesamte Einsatzgemisch, eingesetzt.

Das erfindungsgemäße Verfahren wird bei einer Temperatur von 400 bis 600°C, bevorzugt 420 bis 520°C, und bei einem Druck von 0,5 bis 10 bar, vorzugsweise 1 bis 3 bar, durchgeführt.

Im erfindungsgemäßen Verfahren wird eine Verweilzeit von 0,2 bis 20, bevorzugt von 1 bis 10, besonders bevorzugt von 2 bis 6 Sekunden, eingestellt. Hierzu wird eine Menge Katalysator eingesetzt, die einer Menge von 180 bis 18 000, bevorzugt 360 bis 3600, besonders bevorzugt 600 bis 1800 Vol.-Teilen stündlich durchgesetzten Gemisches pro Schüttvolumenteil-Katalysator entspricht.

Die genannte Verweilzeit des eingespeisten umzusetzenden Gemisches an dem Katalysator bedeutet eine Gas/Raum-Geschwindigkeit (GHSV = Gas Hourly Space Velocity) von etwa 180 bis 18 000, bevorzugt 360 bis 3600, besonders bevorzugt 600 bis 1800 Vol.-Teilen Gasgemisch pro Vol.-Teil Katalysatorschüttung pro Stunden.

Im erfindungsgemäßen Verfahren wird die Umwandlung von n-Alkenen in i-Alkene, bevorzugt von n-Butenen in i-Buten bis nahe an die Einstellung des thermodynamischen Gleichgewichts erreicht. Dieses Gleichgewicht liegt bei den Butenen zwischen 400 und 500°C etwa bei 36—40 Gew.-% für den Fall, daß man das reine System der n- und i-Butene betrachtet. Dieses Gleichgewicht wid bei einem einmaligen Kontakt des erfindungsgemäß einzusetzenden Gemisches mit dem erfindungsgemäß einzusetzenden Katalysator häufig nicht erreicht. In einer besonderen Verfahrensvariante kann jedoch der das Katalysatorbett verlassene Produktstrom aufgeteilt werden, wobei nur ein Teil unmittelbar der Aufarbeitung zugeführt wird, während der andere Teil zusammen mit dem Ausgangskohlenwasserstoffgemisch erneut über das Katalysatorbett gefahren wird. Diese Aufteilung des Produktstromes zur Rückführung kann in weiten Grenzen variieren, beispielsweise zwischen den Verhältnissen 1 : 9 bis 9 : 1 aufgearbeitetes bzw. zurückgeführtes Material. Eine hohe Rückführungsrate bedeutet hierbei einen geringeren Durchsatz, bezogen auf eine konstante Katalysatormenge und konstante sonstige Reaktionsbedingungen, bringt aber eine gewünschte Verschiebung des Komponentenspektrums zugunsten des i-Alkens, beispielsweise des i-Butens bis nahe an das thermodynamische Gleichgewicht. Eine Entscheidung für die Höhe der Rückführungsrate wird bei konstanten sonstigen Verfahrensparametern vor allen Dingen von der Zusammensetzung des zur Verfügung stehenden Ausgangskohlenwasserstoffgemisches abhängen. Mit den erfindungsgemäßen Katalysatoren kann aber im allgemeinen auf eine hohe Rückführungsrate verzichtet werden. Dies kann jedoch durch einfache Vorversuche optimiert werden.

Aus dem obengenannten typischen $C_4$-Schnitt, der nach der Entfernung des i-Butens in einem der genannten Verfahren übrig bleibt und die folgende Zusammensetzung hat: 0 bis 2 Gew.-% i-Buten, 60 bis 80 Gew.-% n-Butene, 10 bis 30 Gew.-% n-Butan und 5 bis 10 Gew.-% i-Butan, kann beispielsweise erfindungsgemäß ein Kohlenwasserstoffgemisch folgender Zusammensetzung erhalten werden: etwa 25 Gew.-% i-Buten, etwa 41 Gew.-% n-Butene, etwa 25 Gew.-% n-Butan/i-Butan, etwa 1 Gew.-% $C_3$-Kohlenwasserstoffe und etwa 8 Gew.-% $C_5$- und höhere Kohlenwasserstoffe. Dieses Reaktionsprodukt kann beispielsweise direkt einer erneuten Reaktion zur Herstellung von Methyl-tert.-butylether, von Oligomeren des Isobutens oder einer Extraktion des Isobutens mit selektiven Lösungsmitteln oder mit Schwefelsäure zugeführt werden. Das nach Abtrennung des i-Butens verbleibende Restgas kann sodann erneut im erfindungsgemäßen Verfahren eingesetzt werden.

Die Erfindung betrifft weiterhin einen Katalysator, mit dem das erfindungsgemäße Verfahren der Umwandlung von n-Alkene in i-Alkene durchgeführt wird. Dieser Katalysator besteht aus einem speziell aktivierten $Al_2O_3$ und/oder $SiO_2$, das mit 0,1 bis 20 Gew.-%, bezogen auf die Menge des $Al_2O_3$ und/oder $SiO_2$, von Halogenverbindungen von Elementen der dritten bis fünften Hauptgruppe und zweiten und achten Nebengruppe des Periodensystems (Mendelejew) und/oder Halogenwasserstoffsäuren dotiert ist, wobei die spezielle Aktivierung darin besteht, daß ein Aluminiumhydroxid und/oder Aluminiumoxidhydrat und/oder Kieselsäuregel mit einem Gehalt an flüchtigen Ammoniumsalzen vor der Dotierung unter Austreibung der flüchtigen Ammoniumsalze zu $Al_2O_3$ und/oder $SiO_2$ kalziniert wird.

Als Halogenverbindungen der genannten Elemente seien beispielsweise genannt: Aluminiumfluorid, Aluminiumchlorid, Aluminiumbromid, Borfluorid, Borchlorid, Titanylchlorid, Germaniumchlorid, Germaniumfluorid, Zinnchlorid, Arsenfluorid, Arsenchlorid, Antimonfluorid, Antimonchlorid, Antimonbromid, Bismutchlorid, Bismutychlorid, Zinkchlorid, Zinkbromid, Cadmiumfluorid, Cadmiumchlorid, Lanthanfluorid, Lanthanchlorid, Lanthanbromid, Vanadylchlorid, Wolframoxichlorid, Eisenfluorid, Eisenchlorid, Eisenbromid, Kobaltchlorid, Nickelchlorid.

Auch Halogenwasserstoffsäuren können sowohl allein als auch in Verbindung mit den obengenannten Halogenverbindungen als Dotierung verwendet werden, beispielsweise Fluorwasserstoff, Chlorwasserstoff, Bromwasserstoff, Jodwasserstoff oder Gemische hieraus.

Die genannten Halogenverbindungen und Halogenwasserstoffsäuren können sowohl als Einzelverbindung als auch als Gemische mehrerer von ihnen zur Dotierung eingesetzt werden.

Bevorzugt als Halogenverbindung und/oder Halogenwasserstoff seien genannt: Aluminiumchlorid, Borfluorid, Antimon-(III)-fluorid, Vanadylchlorid, Titanylchlorid, Bismutylchlorid, Chlorwasserstoff und Bromwasserstoff. Besonders bevorzugt seien genannt: Borfluorid, Antimon-(III)-fluorid und Chlorwasserstoff. Ganz besonders bevorzugt ist Chlorwasserstoff.

Diese Halogenverbindungen und/oder Halogenwasserstoffsäuren werden in einer Menge von 0,1 bis 20 Gew.-%, bevorzugt 0,5 bis 15 Gew.-%, besonders bevorzugt 1 bis 10 Gew.-%, bezogen auf das Gewicht des $Al_2O_3$ und/oder $SiO_2$, zur Dotierung angewendet.

Das $Al_2O_3$ und/oder $SiO_2$ für den erfindungsgemäßen Katalysator wird vor der Dotierung mit den genannten Halogenverbindungen speziell aktiviert. Diese spezielle Aktivierung besteht darin, daß bei der Herstellung des noch nicht dotierten $Al_2O_3$ und/oder $SiO_2$ durch Kalzinieren von Aluminiumhydroxid und/oder Aluminiumoxidhydrat und/oder Kieselsäuregel ein flüchtiges Ammoniumsalz, das in dem Aluminiumhydroxid und/oder Aluminiumoxidhydrat und/oder Kieselsäuregel absorbtiv gebunden war, ausgetrieben wird und dadurch eine besonders aktive Struktur des kalzinierten $Al_2O_3$ und/oder $SiO_2$ hervorruft. Die Struktur des $Al_2O_3$ und/oder $SiO_2$ und seine Dotierung sind daher eng mit dem Herstellungsverfahren für diesen Katalysator verknüpft. $Al_2O_3$ und $SiO_2$ können einzeln oder als Gemisch eingesetzt werden. Von den zu dotierenden $Al_2O_3$ und/oder $SiO_2$ ist das $Al_2O_3$ bevorzugt. Entsprechend wird die spezielle Aktivierung von Aluminiumhydroxid und/oder Aluminiumoxidhydrat bevorzugt.

Die Erfindung betrifft daher weiterhin ein Verfahren zur Herstellung eines speziell aktivierten und mit Halogenverbindungen dotierten $Al_2O_3$- und/oder $SiO_2$-Katalysators, das dadurch gekennzeichnet ist, daß Aluminiumhydroxid und/oder Aluminiumoxidhydrat und/oder Kieselsäuregel mit überschüssiger Lösung von flüchtigen Ammoniumsalzen zur Absorbtion dieser Salze behandelt wird, das so behandelte Aluminiumhydroxid und/oder Aluminiumoxidhydrat und/oder Kieselsäuregel von der restlichen Lösung dieser Salze abgetrennt wird, bei 60 bis 200°C getrocknet und bei 400 bis 800°C unter Austreibung der flüchtigen Ammoniumsalze und unter Ausbildung von $Al_2O_3$ und/oder $SiO_2$ kalziniert wird und danach mit 0,1 bis 20 Gew.-%, bezogen auf die Menge des $Al_2O_3$ und/oder $SiO_2$, von Halogenverbindungen von Elementen der dritten bis fünften Hauptgruppe und zweiten bis achten Nebengruppe des Periodensystems (Mendelejew) und/oder Halogenwasserstoffsäuren dotiert wird.

Als flüchtiges Ammoniumsalz sei hierbei beispielsweise ein Salz des Ammoniaks oder eines niederen primären, sekundären oder tertiären aliphatischen Amins genannt, wie des Methylamin, Dimethylamin, Trimethylamin, Ethylamin, Diethylamin oder Triethylamin. Bevorzugt werden Salze des Ammoniaks eingesetzt. Als Anionen seien anorganische oder organische Anionen genannt, wie Fluorid, Chlorid, Bromid, Jodid, Nitrat, Sulfat, Phosphat, Carbonat, Hydrogencarbonat, Hydrogensulfat, Hydrogenphosphat, Acetat, Propionat, Formiat, Oxalat oder Benzoat. Bevorzugt werden Salze anorganischer Säuren, besonders bevorzugt der leichter flüchtigen anorganischen Säuren, wie das Fluorid, Chlorid, Bromid, Jodid oder Nitrat eingesetzt. In ganz besonders bevorzugter Weise wird Ammoniumnitrat eingesetzt.

Ein solches flüchtiges Ammoniumsalz wird beispielsweise in einer etwa 5- bis 20%igen Lösung in einem geeigneten Lösungsmittel, wie Wasser, Methanol oder Ethanol, bevorzugt Wasser, zu dem Aluminiumhydroxid und/oder Aluminiumoxidhydrat und/oder Kieselsäuregel gegeben und etwa eine halbe bis 6 Stunden damit unter Rühren in Kontakt gebracht, um die Absorption durch die Aluminiumverbindung und/oder das Kieselsäuregel möglichst zu vervollständigen. Die absorptiv gebundene Menge an Ammoniumsalz beträgt etwa 10 bis 70 Gew.-%, bezogen auf das getrocknete Aluminiumhydroxid und/oder Aluminiumoxidhydrat und/oder das Kieselsäuregel.

Im Anschluß hieran wird das Aluminiumhydroxid und/oder Aluminiumoxidhydrat und/oder das Kieselsäuregel mit dem absorptiv gebundenen Ammoniumsalz von der überschüssigen Lösung, beispielsweise durch Filtration oder Zentrifugieren, abgetrennt und bei einer Temperatur von 60 bis 200° C, bevorzugt 100 bis 160° C, gegebenenfalls unter Anlegen von Vakuum, getrocknet. Das getrocknete Material wird sodann anschließend bei 400 bis 800° C, bevorzugt 500 bis 650° C, unter Austreibung der flüchtigen Ammoniumsalze und unter Ausbildung des $Al_2O_3$ und/oder $SiO_2$ kalziniert. Das Trocknen und das Kalzinieren kann zweckmäßigerweise in einem Umluftofen durchgeführt werden, der sowohl mit Luft als auch mit Stickstoff, Argon oder $CO_2$ beschickt werden kann.

In einer besonderen Verfahrensvariante für den Fall, daß der zu dotierende und speziell aktivierte Stoff für den Katalysator $Al_2O_3$ ist, wird nicht von einem separat hergestellten Aluminiumhydroxid und/oder Aluminiumoxidhydrat ausgegangen, sondern von einem frisch gefällten Aluminiumhydroxid. Als flüchtiges Ammoniumsalz wird hierbei kein gesondert zugesetztes benutzt, sondern das Salz genommen, das sich aus dem als Fällungsmittel verwendeten wäßrigen Ammoniak und dem Anion des eingesetzten Aluminiumsalzes bildet. Beispielsweise wird Aluminiumchlorid, Aluminiumbromid oder Aluminiumnitrat mit konzentriertem oder verdünntem wäßrigem Ammoniak bei 0 bis 90° C, bevorzugt bei Zimmertemperatur, gefällt und das dabei entstehende Aluminiumhydroxid noch einige Zeit, beispielsweise 0,5 bis 6 Stunden, im Kontakt mit der Fällungslösung gelassen, die das gebildete Ammoniumchlorid, Ammoniumbromid oder Ammoniumnitrat enthält. Das Aluminiumhydroxid bindet hierbei absorptiv dieses vorhandene Ammoniumsalz, es wird anschließend abfiltriert oder abzentrifugiert und ohne einen Auswaschvorgang sofort der oben beschriebenen Trocknung und anschließend der Kalzinierung zugeführt.

Das durch die simultane Kalzinierung und Austreibung der flüchtigen Ammoniumsalze gebildete $Al_2O_3$ und/oder $SiO_2$ liegt sodann in einer besonders aktiven Form vor, die anschließend mit den obengenannten Mengen der beschriebenen Halogenverbindungen dotiert werden kann. Hierzu wird das auf eine Korngröße von 0,01 bis 20 mm $\varnothing$ gebrachte $Al_2O_3$ und/oder $SiO_2$ mit der Halogenverbindung, gelöst in einem geeigneten Lösungsmittel, in Kontakt gebracht und anschließend nochmals kalziniert. Als Lösungsmittel für die beschriebenen Halogenverbindungen und/oder Halogenwasserstoffsäuren kommt in den allermeisten Fällen Wasser in Frage. Für den Fall, daß hydrolyseempfindliche Halogenverbindungen eingesetzt werden sollen, kann zur Zurückdrängung dieser Hydrolyse ein Zusatz der entsprechenden Halogenwasserstoffsäure zum Wasser vorgenommen werden. Bei wasserempfindlichen Halogenverbindungen kann weiterhin ein polares Lösungsmittel, wie Aceton oder Acetonitril angewendet werden. Zur gleichmäßigen Dotierung des $Al_2O_3$ und/oder $SiO_2$ ist es zweckmäßig, ein solches Volumen der Lösung aus dem Lösungsmittel und der beschriebene Halogenverbindung anzuwenden, das etwa dem nach einer geeigneten Methode bestimmten Porenvolumen des $Al_2O_3$ und/oder $SiO_2$ entspricht. Hierzu sei beispielsweise eine Menge von 80 bis 300, bevorzugt 100 bis 200 Vol.-% Lösung, bezogen auf das Porenvolumen des $Al_2O_3$ und/oder $SiO_2$, genannt.

Der dotierte Katalysator wird sodann für etwa 1 bis 10, bevorzugt 2 bis 8 Stunden, bei etwa 400 bis 600° C, bevorzugt 420 bis 500° C, kalziniert. Er wird sodann auf eine Korngröße von 0,05 bis 10 mm gemahlen und ist danach gebrauchsfertig.

Es wurde gefunden, daß der dotierte $Al_2O_3$- und/oder $SiO_2$-Katalysator nach längerer Gebrauchsdauer und/oder nachlassender Umwandlungsaktivität durch Erhitzen bei 400 bis 800° C, bevorzugt 550 bis 650° C, auf seine ursprüngliche Aktivität regeneriert werden kann. Dieses Regenerieren wird in einer sauerstoffhaltigen Atmosphäre die neben Sauerstoff auch Inertgase, wie Stickstoff, Wasserdampf oder andere Gase, wie Edelgase, CO oder $CO_2$ enthalten kann, unter Zusatz von halogenierten Kohlenwasserstoffen, wie Tetrachlorkohlenstoff, Chloroform, chlorierten Ethanen, Dichlorpropan, Fluorchlorethan und ähnlichen, durchgeführt. Der Druck im Regenerierungsreaktor ist nicht erfindungswesentlich und kann in weiten Grenzen schwanken, beispielsweise von 0,5 bis 20 bar, bevorzugt 1 bis 10 bar, besonders bevorzugt 2—5 bar. Der zu regenerierende Katalysator wird 0,01 bis 10, bevorzugt 0,1 bis 5, besonders bevorzugt 0,2 bis 3 Stunden unter diesen Bedingungen belassen. Die genannten halogenierten Kohlenwasserstoffe werden bei diskontinuierlicher Führung des Regenerierungsprozesses in einer Menge von beispielsweise 0,1 bis 1% und bei kontinuierlicher Führung des Regenerierungsprozesses in einer Menge von 1 bis 100 ppm, bezogen auf das gesamte Einsatzgemisch, bestehend aus Katalysator und der Regenerierungsatmosphäre, eingesetzt.

Das Verhältnis des zu regenerierenden Katalysators und des genannten Mediums als Regenerierungsatmosphäre kann in weiten Grenzen schwanken. Für die Regeneration seien beispielsweise 200—5000 Liter dieser Atmosphäre pro Kilogramm des zu regenerierenden Katalysators genannt. Der Sauerstoffgehalt der Regenerierungsatmosphäre beträgt beispielsweise 5 bis 50 Vol.-% $O_2$; bevorzugt wird Luft eingesetzt.

Es kann zweckmäßig sein, vor dem Regenerierungsschritt noch einmal eine Dotierung mit Halogenverbindungen, wie oben ausgeführt, durchzuführen. Diese nochmalige Dotierung ist jedoch in vielen Fällen nicht erforderlich.

Der regenerierte Katalysator ist sodann wieder vollwertig für die Isomerisierungsreaktion einsetzbar.

Im erfindungsgemäßen Verfahren wird der Katalysator im bewegten Katalysatorbett angewandt. Als bewegtes Katalysatorbett sei beispielsweise das Wirbelbett oder das Fließbett genannt. Bevorzugt ist hierbei die Anwendung des Katalysators im Fließbett.

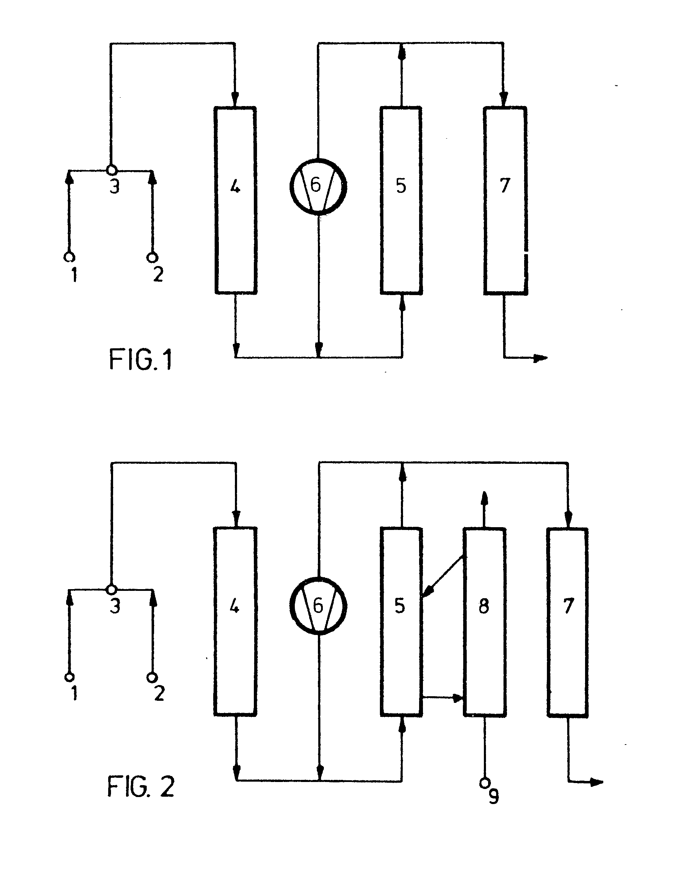

In Fig. 1 ist beispielsweise ein Schema für die mögliche Durchführung des erfindungsgemäßen Verfahrens angegeben:

1) bedeutet hierin die Zufuhr des n-Alken-haltigen Kohlenwasserstoffstromes, 2) bedeutet hierin die Zufuhr des Wasserstromes, 3) bedeutet hierin eine Misch- und Dosiereinrichtung, 4) bedeutet hierin einen Vorwärmer, 5) bedeutet hierin den Reaktor mit dem erfindungsgemäß einzusetzenden Katalysator. Dieser kann im Reaktor sowohl als Festbett als auch als bewegtes Bett, bevorzugt als bewegtes Bett, angeordnet sein. 6) bedeutet eine Umwälzpumpe, durch die ein Teil des am Kopf von 5) entnommenen Reaktionsproduktes in den unteren Teil von 5) wieder zurückgeführt wird, 7) bedeutet einen Kühler für die heißen Reaktionsprodukte, die danach zur weiteren Aufarbeitung oder Verarbeitung abgeführt werden.

Es ist bekannt, daß viele Reaktionen im Festbett mit hoher Ausbeute und guter Selektivität ablaufen. Um die Reaktion von n-Alkenen zu i-Alkenen, insbesondere von n-Butenen zu i-Buten, mit ausreichender Geschwindigkeit ablaufen zu lassen, muß die Isomerisierung bei hoher Temperatur, beispielsweise zwischen 400 bis 600°C, und bei niedrigen Drucken durchgeführt werden. Entsprechend den gewählten Reaktionsbedingungen ist die Ausbeute beispielsweise an i-Buten aus diesem Isomerisierungsprozeß thermodynamisch begrenzt, so daß die in der Literatur bekannten Verfahren besonders durch die Anwendung des Festbettes auf eine möglichst hohe Ausbeute und Selektivität abzielen. Hierbei wurden die oben beschriebenen Nachteile, d. h. der schnelle Aktivitäts- und Selektivitätsverlust des Katalysators durch die unerwünschte Verkokungsreaktion, die Oligomerenbildung und Crackreaktionen, bewußt in Kauf genommen.

Ein weiterer Nachteil für eine technische Anwendung der Isomerisierung in einer Festbettreaktion ist die kontinuierlich wechselnde Produktzusammensetzung und das erforderliche Umschalten von einem Katalysatorbett auf das nächste zur Ermöglichung einer Regenerierungsperiode.

Es ist daher von besonderem Vorteil, daß bei Verwendung des oben beschriebenen Katalysators die Skelettisomerisierung von n-Alkenen zu i-Alkenen mit hoher Ausbeute und hervorragender Selektivität ohne die angeführten Nachteile erzielt werden kann, wenn man die Isomerisierung in einem bewegten Katalysatorbett durchführt.

Von ganz besonderen Vorteil ist hierbei eine Verfahrensvariante, bei der der Katalysator ohne Verfahrensunterbrechung ganz oder teilweise durch neuen und/oder regenerierten Katalysator ersetzt wird.

Hierzu wird der im Verfahren befindliche Katalysator in dem Maße aus dem Reaktor entnommen, wie ein neuer und/oder regenerierter Katalysator in den Reaktor eingeführt wird. Das Ausschleusen des im Verfahren befindlichen Katalysators und der Ersatz durch neuen und/oder regenerierten Katalysator kann weiterhin absatzweise oder kontinuierlich erfolgen. Bei absatzweiser Entnahme und Ersatz des Katalysators kann beispielsweise der im Verfahren befindliche Katalysator ganz oder teilweise bei gleichzeitigem Ersatz durch frischen Katalysator aus dem Reaktor entnommen werden, wonach die Isomerisierungsreaktion dann ohne Katalysatoraustausch weiter betrieben wird, bis nach einer geeigneten Zeit ein erneutes Ausschleusen und Ersetzen des Katalysators vorgenommen wird. Bevorzugt ist das Arbeiten mit kontinuierlicher Ausschleusung und Ersatz des Katalysators. Allgemein kann so verfahren werden, daß die gesamte Katalysatormenge diskontinuierlich oder kontinuierlich aus dem bewegten Katalysatorbett innerhalb einer Zeit von 5 Tagen bis zu 30 Tagen, bevorzugt 7 bis 20 Tagen, je einmal vollständig ausgetauscht und ersetzt worden ist. Bei kontinuierlichem Austausch und Ersatz entspricht dies beispielsweise etwa 1—0,01% der Gesamtmenge der Katalysatorfüllung pro Stunde.

Der Regenerierungsprozeß selbst kann sowohl diskontinuierlich als auch kontinuierlich ausgeführt werden. Eine bevorzugte Ausführungsform stellt die Kombination des kontinuierlichen Ausschleusens und Ersatzes von Katalysator aus dem bewegten Katalysatorbett mit einer kontinuierlichen Regenerierung dieses Katalysators in einem besonderen, von der Isomerisierungsreaktion getrennten Regene-

0 054 855

rierungsreaktor dar.

Selbstverständlich ist es erfindungsgemäß möglich, anstelle des ausgeschleusten Katalysators vollständig neuen Katalysator in das bewegte Katalysatorbett der Isomerisierungsreaktion einzuführen. In bevorzugter Weise wird jedoch der regenerierte Katalysator zurückgeführt. Neuer Katalysator ist im allgemeinen nur erforderlich, um geringe Katalysatorverluste, beispielsweise als Flugstaub, zu ersetzen.

Die Anwendung des erfindungsgemäßen Verfahrens des bewegten Katalysatorbettes und der separat ohne Verfahrensunterbrechung möglichen Katalysatorregenerierung erlaubt es, während einer unbegrenzten Betriebszeit und unter Steuerung der zur Regenerierung ausgeschleusten bzw. aus der Regenerierung wieder zurückgeführten Katalysatormenge ein gleichmäßig zusammengesetztes Reaktionsprodukt bei gleichmäßiger Ausbeute an i-Alken und konstanten Betriebsbedingungen zu erzeugen. Es ist also nicht erforderlich, gemäß dem Stand der Technik wechselnde Produktzusammensetzung und das Ein- und Abschalten verschiedener Reaktionssysteme in Kauf zu nehmen. So müßte man bei einem Festbettverfahren, um ein dem Wirbelschichtverfahren annähernd vergleichbares Ergebnis zu erreichen, nach so kurzen Zeitabständen wie jeweils nach 12—24 Stunden den verkokten Katalysator abbrennen und regenerieren. Neben den Schwierigkeiten der wechselnden und unterbrochenen Verfahrensführung und der daraus resultierenden schwankenden Produktzusammensetzung bedeutet jedes Abbrennen von Koks auf dem Katalysator eine Belastung dieses Katalysators, welche sich bei häufiger Anwendung negativ auf die Aktivität und Lebensdauer des Katalysators auswirkt. Weiterhin ist es erfindungsgemäß möglich, bei konstanter Aufrechterhaltung des Isomerisierungsprozesses, Katalysatoren durch andere zu ersetzen, zu dotieren, aufzufrischen und durch Abrieb zerstörte Katalysatormengen zu ersetzen bzw. geringe Aktivitätsverluste durch das Einschleusen von frischem Katalysator auszugleichen. Alle beschriebenen Operationen mit dem Katalysator finden außerhalb des Isomerisierungsreaktors statt und führen daher nicht zu einer Verunreinigung der zu isomerisierenden Stoffe.

Fig. 2 zeigt eine mögliche Ausführungsform der Isomerisierungsreaktion bei Anwendung des Katalysators im bewegten Katalysatorbett und kontinuierlicher Regeneration des Katalysators. Hierin haben (1) bis (7) gleiche Bedeutung wie in Fig. 1.

Zusätzlich bedeuten (8) einen Regenerierungsreaktor und (9) die Zuführung der für die Regenerierungsatmosphäre benötigten und oben beschriebenen Stoffe.

Fig. 2 zeigt ein Beispiel für die Ausführung des erfindungsgemäßen Verfahrens, wenn in bevorzugter Weise aus dem bewegten Katalysatorbett im Reaktor (5) in kontinuierlicher Weise Katalysator entnommen wird, kontinuierlich in den Regenerierungskatalysator (8) eingespeist wird und in kontinuierlicher Weise aus diesem Regenerierungskatalysator wieder in das bewegte Katalysatorbett im Reaktor (5) zurückgeführt wird.

Im Gegensatz zu früher beschriebenen Versuchen der Skelettisomerisierung von Olefinen läßt sich das erfindungsgemäße Verfahren bei hohen Standzeiten des Katalysators mit hoher Aktivität und hoher Selektivität ohne Koksablagerung und ohne Oligomerenbildung mit hoher Ausbeute von i-Alkenen durchführen. Die hohe Katalysatorstandzeit läßt sich durch die beschriebenen Regenerierungsmaßnahmen weiter verbessern, so daß das erfindungsgemäße Verfahren auch äußerst wirtschaftlich durchgeführt werden kann. Es ist überraschend, daß die Anwesenheit von flüchtigen Ammoniumsalzen zu Beginn der Kalzinierung des noch nicht dotierten Aluminiumhydroxids und/oder Aluminiumoxidhydrats und/oder Kieselsäuregels, die während der Ausbildung des $Al_2O_3$ und/oder $SiO_2$ ausgetrieben werden, dem $Al_2O_3$ und/oder $SiO_2$ solche vorteilhaften Eigenschaften geben, daß die übrigen erfindungsgemäßen Maßnahmen, wie die Dotierung und die Zumischung von Wasserstoff zu den n-Alken-haltigen Ausgangsprodukten ihre volle Wirksamkeit entfalten.

Beispiel 1

100 g $Al(NO_3)_3 \cdot 9 H_2O$ werden in 500 ml destilliertem Wasser gelöst und mit 60 ml konzentriertem wäßrigen Ammoniak innerhalb von 2 Stunden gefällt. Das ausgefällte Aluminiumhydroxid wird noch 2 Stunden gerührt, abfiltriert und 24 Stunden bei 150°C getrocknet. Die trockene Masse wird dann 1 Stunde unter Stickstoff bei 600°C erhitzt. Auf diesen so hergestellten $Al_2O_3$-Träger wird eine Lösung von 0,75 g $SbF_3$ in 10 ml destilliertem Wasser aufgebracht und 2 Stunden bei 150°C getrocknet und anschließend bei 450°C 4 bis 5 Stunden kalziniert. Der so hergestellte Katalysator wird danach auf die erforderliche Korngröße durch Zerkleinern und fraktioniertes Aussieben gebracht.

Beispiel 2

Zur Durchführung wird die in Fig. 1 schematisch dargestellte Apparatur benutzt. Über die Dosiervorrichtung (3) wird ein gasförmiges Gemisch aus Buten-1 (1) und Wasserstoff (2) im Volumenverhältnis von 1 : 1,5 über den Vorwärmer (4) in den Reaktor (5) gefahren. Ein Teil des Reaktionsproduktes wird über die Kreisgaspumpe (6) dem Reaktor (5) wieder zugeführt, in einem Maße, daß das Volumenver-

7

hältnis zwischen Einsatzmenge und Kreisgasmenge 1 : 1 beträgt.

Als Katalysator wurde ein nach Beispiel 1 hergestelltes, jedoch mit 9,1 Gew.-% BiOCl dotiertes Aluminiumoxid eingesetzt. Die Reaktionstemperatur betrug 480°C, der Druck 1 bar, die Gasraumgeschwindigkeit GHSV (Gas hourly space velocity) 1500 $h^{-1}$, entsprechend einer mittleren Verweilzeit von 2,4 Sekunden. Das hinter dem Kühler (7) ausgeschleuste flüssige Reaktionsprodukt hatte folgende Zusammensetzung:

| | |
|---|---|
| $C_3$-Kohlenwasserstoff | 1,6 Gew.-% |
| i-Buten | 33,0 Gew.-% |
| n-Butene | 50,6 Gew.-% |
| Butan, i-Butan | 3,7 Gew.-% |
| $C_5$-Kohlenwasserstoffe und höhere | 11,1 Gew.-% |
| | 100,0 Gew.-% |

## Beispiel 3

Entsprechend Beispiel 2 wird in einer Apparatur nach Fig. 1 ein gasförmiges Gemisch aus einem Kohlenwasserstoffstrom (1), bestehend aus 0,5 Gew.-% i-Buten, 76 Gew.-% n-Butene und 23,5 Gew.-% Butane, mit Wasserstoff (2) im Volumenverhältnis von 1 : 1,5 über den Vorwärmer (4) in den Reaktor (5) gefahren. Als Katalysator wurde ein nach Beispiel 1 hergestelltes, jedoch mit 1,5 Gew.-% $BF_3$ dotiertes Aluminiumoxid eingesetzt. Das Kreislaufverhältnis betrug 1 :1, die Reaktionstemperatur 450°C und die Gasraumgeschwindigkeit GHSV 1500 $h^{-1}$, entsprechend einer mittleren Verweilzeit von 2,4 Sekunden. Das hinter dem Kühler (7) ausgeschleuste flüssige Reaktionsprodukt hat folgende Zusammensetzung:

| | |
|---|---|
| $C_3$-Kohlenwasserstoffe | 1,7 Gew.-% |
| i-Buten | 23,1 Gew.-% |
| n-Butene | 38,6 Gew.-% |
| Butan, i-Butan | 25,2 Gew.-% |
| $C_5$-Kohlenwasserstoffe und höhere | 11,4 Gew.-% |
| | 100,0 Gew.-% |

## Beispiele 4—6

Ein handelsübliches Filtrol Grade 13 entsprechend US 3 663 453 sowie ein nach Beispiel 1 hergestelltes $Al_2O_3$ wurden durch Zerkleinern und Sieben auf die gleiche Korngröße gebracht, mit 9,1 Gew.-% $ZrOCl_2 \cdot 8 H_2O$ dotiert und als Katalysator eingesetzt. Weiterhin wurde ein $Al_2O_3$ nach Beispiel 1 aber ohne Dotierung zum Vergleich herangezogen.

Entsprechend Beispiel 2 wurde ein gasförmiger $C_4$-Strom mit Wasserstoff im Verhältnis von 1 : 1,5 über den Vorwärmer in den Reaktor gefahren. Das Verhältnis von Kreisgas zu Einsatzmaterial betrug 1 :1, die Reaktionstemperatur 480°C und die Gasraumgeschwindigkeit GHSV 1500 $h^{-1}$, entsprechend einer mittleren Verweilzeit von 2,4 sec. Das hinter dem Kühler ausgeschleuste flüssige Reaktionsprodukt hatte folgende Zusammensetzung:

| | Beispiele | | |
| --- | --- | --- | --- |
| | 4 | 5 | 6 |
| | Katalysator | | |
| | Filtrol Grade 13 | $Al_2O_3$ nach | $Al_2O_3 + ZrOCl_2$ |
| | + $ZrOCl_2$ nach | Beispiel 1 | entsprechend |
| | US 3 663 453 | ohne Dotierung | Beispiel 1 |
| $C_3$ | 1,2 | 0,6 | 2,0 |
| n-Butan/Isobutan | 23,6 | 22,5 | 26,2 |
| Isobuten | 9,8 | 12,4 | 24,2 |
| Buten-1/Buten-2 | 61,6 | 62,3 | 39,7 |
| $C_5$ und Höhere | 3,8 | 2,2 | 7,9 |
| Umsatz | 18,0 | 17,0 | 47,1 |
| Selektivität | 52,2 | 75,4 | 60,6 |

Der Katalysator in Versuch 4 verkokte nach etwa 1 Stunde und zeigte dabei einen starken Verfall seiner Aktivität und eine starke Verschlechterung des anfänglichen i-Buten-Wertes, während der Versuch 6 nach 72 Stunden ohne erkennbare Änderung der Aktivitäts- und Ausbeutewerte abgestellt wurde.

## Beispiel 7

Entsprechend Beispiel 2 und 3 wird in einer Apparatur nach Fig. 1 ein gasförmiges Gemisch aus einem Kohlenwasserstoffstrom (1), bestehend aus 1 Gew.-% i-Buten, 75 Gew.-% n-Buten und 24 Gew.-% Butane, mit Wasserstoff (2) im Volumenverhältnis von 1 : 1,5 über den Vorwärmer (4) in den Reaktor (5) gefahren. Als Katalysator wurde ein nach Beispiel 1 hergestelltes, jedoch mit 15 Gew.-% Chlorwasserstoff dotiertes Aluminiumoxid eingesetzt. Das Kreislaufverhältnis betrug 1 : 1, die Reaktionstemperatur 450° C und die Gasraumgeschwindigkeit GHSV 1500 $h^{-1}$, entsprechend einer mittleren Verweilzeit von 2,4 Sekunden. Das hinter dem Kühler (7) ausgeschleuste flüssige Reaktionsprodukt hat folgende Zusammensetzung:

| | |
| --- | --- |
| $C_3$-Kohlenwasserstoffe | 1,9 Gew.-% |
| i-Buten | 25,1 Gew.-% |
| n-Butene | 41,3 Gew.-% |
| Butan, i-Butan | 23,6 Gew.-% |
| $C_5$-Kohlenwasserstoffe und höhere | 8,1 Gew.-% |
| | 100,0 Gew.-% |

## Beispiel 8 (zum Vergleich)

In diesem Beispiel wurde die Apparatur wie in Fig. 1 mit Anordnung des Katalysators im festen Katalysatorbett betrieben. Als Katalysator wurde ein nach Beispiel 1 hergestelltes, jedoch mit 15 Gew.-% Chlorwasserstoff dotiertes Aluminiumoxid eingesetzt. Ein gasförmiges Gemisch aus einem Kohlenwasserstoff (1), bestehend aus 0,5 Gew.-% i-Buten, 71 Gew.-% n-Butenen, 28 Gew.-% Butanen und 0,5 Gew.-% anderen Bestandteilen, wird mit Wasserstoff (2) im Vol.-Verhältnis 1 : 1,5 gemischt und über den Vorwärmer (4) in den Festbettreaktor (5) gefahren. Die Reaktionstemperatur beträgt 450° C, die GHSV 1500 $h^{-1}$ und das Kreislaufverhältnis 1 : 1 (Vol./Vol.).

Das hinter dem Kühler (7) ausgeschleuste flüssige Reaktionsprodukt zeigt im Laufe der Reaktionsdauer folgende Zusammensetzungen:

Tabelle I (Beispiel 8)

| | Reaktionsdauer in h | | | regeneriert |
| | nach 10 h | nach 20 h | nach 45 h | nach 50 h |
|---|---|---|---|---|
| Komponenten in Gew.-% | | | | |
| $C_3$-KW | 1,5 | 1,3 | 0,8 | 0,8 |
| i-Buten | 25,1 | 23,5 | 19,0 | 21,5 |
| n-Butene | 39,7 | 43,6 | 49,1 | 45,0 |
| Butan, i-Butan | 28,9 | 27,7 | 27,9 | 28,9 |
| $C_5$-KW und höhere | 4,8 | 3,9 | 3,2 | 3,8 |
| Summe | 100 | 100 | 100 | 100 |

## Beispiel 9

In diesem Beispiel wurde eine Apparatur wie in Fig. 2 mit Anordnung des Katalysator im Wirbelbett betrieben. Als Katalysator wurde ein nach Beispiel 1 hergestelltes, jedoch mit 15 Gew.-% Chlorwasserstoff dotiertes Aluminiumoxid eingesetzt. Ein Kohlenwasserstoffstrom (1), bestehend aus 0,5 Gew.-% i-Buten, 76 Gew.-% n-Butenen und 23,5 Gew.-% Butanen wird im Gemisch mit einem Wasserstoffstrom (2) im Verhältnis 1 : 1,5 umgesetzt. Das Kreislaufverhältnis von ausgeschleuster und rückgeführter Reaktionsgasmenge beträgt 1 : 1, die Reaktionstemperatur 450°C und die Gasraumgeschwindigkeit GHSV 1500 h$^{-1}$. Katalysator wird aus dem Reaktor (5) ausgeschleust und kontinuierlich in dem Reaktor (8) mit Luft (9) regeneriert und in den Wirbelschichtreaktor (5) zurückgeführt. Das hinter dem Kühler (7) ausgeschleuste Reaktionsprodukt hatte im Laufe der Reaktionsdauer folgende Zusammensetzung:

Tabelle II (Beispiel 9)

| | Reaktionsdauer in h | | | |
| | nach 20 h | nach 45 h | nach 85 h | nach 100 h |
|---|---|---|---|---|
| Komponenten in Gew.-% | | | | |
| $C_3$-KW | 1,1 | 1,4 | 1,2 | 0,8 |
| i-Buten | 25,6 | 25,4 | 25,3 | 25,0 |
| n-Butene | 39,0 | 40,4 | 41,6 | 41,5 |
| Butan, i-Butan | 26,0 | 25,8 | 25,5 | 25,0 |
| $C_5$-KW und höhere | 8,3 | 7,0 | 6,4 | 7,7 |
| Summe | 100 | 100 | 100 | 100 |

## Patentansprüche

1. Verfahren zur Herstellung von i-Alkenen durch katalytische Umwandlung von n-Alkenen im Gemisch mit Wasserstoff und gegebenenfalls mit Inertgasen bei erhöhter Temperatur an einem mit Halogenverbindungen von Elementen der dritten bis fünften Hauptgruppe und zweiten bis achten Nebengruppe des Periodensystems (Mendelejew) und/oder Halogenwasserstoffsäuren dotierten $Al_2O_3$- und/oder $SiO_2$-Katalysator, dadurch gekennzeichnet, daß ein speziell aktiviertes $Al_2O_3$ und/ oder $SiO_2$ eingesetzt wird, wobei die spezielle Aktivierung darin besteht, daß ein Aluminiumhydroxid und/oder Aluminiumoxidhydrat und/oder Kieselsäuregel mit einem Gehalt an flüchtigen Ammoniumsalzen vor der Dotierung unter Austreibung der flüchtigen Ammoniumsalze zu $Al_2O_3$ und/oder $SiO_2$ kalziniert wird und daß in einem bewegten Katalysatorbett gearbeitet wird und gegebenenfalls der Katalysator ohne Verfahrensunterbrechung ganz oder teilweise durch neuen und/oder regenerierten Katalysator ersetzt wird.

2. Verfahren nach Anspruch 1, dadurch gekennzeichnet, daß man das Gemisch mit einem in diesem Verfahren bereits eingesetzten, speziell aktivierten und mit den genannten Verfahren nach Halogenverbindungen dotierten $Al_2O_3$ und/oder $SiO_2$-Katalysator in Kontakt bringt, der bei einer Temperatur von 400 bis 800°C in einer sauerstoffhaltigen Atmosphäre, die auch Inertgase enthalten kann, unter Zusatz von halogenierten Kohlenwasserstoffen regeneriert worden ist.

3. Verfahren nach Anspruch 1, dadurch gekennzeichnet, daß bei einer Temperatur von 400 bis 600°C und einer Verweilzeit von 0,2 bis 20 Sekunden gearbeitet wird.

4. Verfahren nach Anspruch 1 bis 3, dadurch gekennzeichnet, daß der Katalysator in einem Fließbett oder in einem Wirbelbett angeordnet ist.

5. Verfahren nach Ansprüchen 1 bis 4, dadurch gekennzeichnet, daß aus dem bewegten Katalysatorbett Teilmengen herausgeschleudert, regeneriert und in das bewegte Katalysatorbett zurückgeführt werden.

6. Verfahren nach Ansprüchen 1 bis 5, dadurch gekennzeichnet, daß die Ausschleusung, Regenerierung und Rückschleusung kontinuierlich erfolgt.

7. Katalysator aus einem $Al_2O_3$ und $SiO_2$, das mit 0,1 bis 20 Gew.-%, bezogen auf die Menge des $Al_2O_3$ und/oder $SiO_2$, von Halogenverbindungen von Elementen der dritten bis fünften Hauptgruppe und zweiten bis achten Nebengruppe des Periodensystems (Mendelejew) und/oder Halogenwasserstoffsäuren dotiert ist, gekennzeichnet durch eine spezielle Aktivierung des $Al_2O_3$ und/oder $SiO_2$, die darin besteht, daß ein Aluminiumhydroxid und/oder Aluminiumoxidhydrat und/oder Kieselsäuregel mit einem Gehalt an flüchtigen Ammoniumsalzen vor der Dotierung unter Austreibung der flüchtigen Ammoniumsalze zu $Al_2O_3$ und/oder $SiO_2$ kalziniert wird.

8. Verfahren zur Herstellung eines speziell aktivierten und mit Halogenverbindungen dotierten $Al_2O_3$ und/oder $SiO_2$-Katalysators, dadurch gekennzeichnet, daß ein Aluminiumhydroxid und/oder Aluminiumoxidhydrat und/oder Kieselsäuregel mit überschüssiger Lösung von flüchtigen Ammoniumsalzen zur Absorption dieser Salze behandelt wird, das so behandelte Aluminiumhydroxid und/oder Aluminiumoxidhydrat und/oder Kieselsäuregel von der restlichen Lösung dieser Salze abgetrennt wird, bei 60 bis 200°C getrocknet und bei 400 bis 800°C unter Austreibung der flüchtigen Ammoniumsalze und unter Ausbildung von $Al_2O_3$ und/oder $SiO_2$ kalziniert wird und danach in bekannter Weise mit 0,1 bis 20 Gew.-%, bezogen auf die Menge des $Al_2O_3$ und/oder $SiO_2$, von Halogenverbindungen von Elementen der dritten bis fünften Hauptgruppe und zweiten bis achten Nebengruppe des Periodensystems (Mendelejew) dotiert wird.

9. Verfahren nach Anspruch 8, dadurch gekennzeichnet, daß als flüchtiges Ammoniumsalz dasjenige gewählt wird, das aus dem Kation des zur Fällung benutzten Ammoniaks und dem Anion des zur Herstellung des Aluminiumhydroxids eingesetzten löslichen Aluminiumsalzes besteht, wobei sich dieses Ammoniumsalz durch absorptive Mitfällung an das frisch gefällte Aluminiumhydroxid anlagert.

## Claims

1. Process for the preparation of isoalkenes by the catalytic conversion of n-alkenes in admixture with hydrogen and, optionally, with inert gases, at an elevated temperature, on an $Al_2O_3$ and/or $SiO_2$ catalyst which is doped with halogen compounds of elements of the third to fifth main groups and second to eighth sub-groups of the periodic system (Mendeléeff) and/or hydrohalic acids, characterised in that a specifically activated $Al_2O_3$ and/or $SiO_2$ is used, the specific activation consisting in calcining an aluminium hydroxide and/or aluminium oxide hydrate and/or silicic-acid gel having a content of volatile ammonium salts, before the doping process, with the expulsion of the volatile ammonium salts, to give $Al_2O_3$ and/or $SiO_2$, and in that the reaction is carried out in a moving catalyst bed and, if appropriate, the catalyst is completely or partly replaced by fresh and/or regenerated catalyst without interrupting the process.

2. Process according to Claim 1, characterised in that the mixture is brought into contact with an $Al_2O_3$ and/or $SiO_2$ catalyst which has already been used in this process and has been specifically activated and doped with the stated halogen compounds, and which has been regenerated at a temperature of 400 to 800°C in an oxygen-containing atmosphere, which can also contain inert gases, with the addition of halogenated hydrocarbons.

3. Process according to Claim 1, characterised in that the reaction is carried out at a temperature of 400 to 600°C and with a dwell time of 0.2 to 20 seconds.

4. Process according to Claims 1 to 3, characterised in that the catalyst is arranged in a fluidised bed or in a turbulent bed.

5. Process according to Claims 1 to 4, characterised in that partial quantities are removed from the moving catalyst bed, regenerated and reintroduced into the moving catalyst bed.

6. Process according to Claims 1 to 5, characterised in that the removal, regeneration and reintroduction are carried out continuously.

7. Catalyst consisting of an $Al_2O_3$ and $SiO_2$ which is doped with 0.1 to 20% by weight, based on the quantity of the $Al_2O_3$ and/or $SiO_2$, of halogen compounds of elements of the third to fifth main groups and second to eighth sub-groups of the periodic system (Mendeléeff) and/or hydrohalic acids, characterised by a specific activation of the $Al_2O_3$ and/or $SiO_2$, which consists in calcining an aluminium hydroxide and/or aluminium oxide hydrate and/or silicic-acid gel having a content of volatile ammonium salts, before the doping process, with the expulsion of the volatile ammonium salts, to give $Al_2O_3$ and/or $SiO_2$.

8. Process for the preparation of a specifically activated $Al_2O_3$ and/or $SiO_2$ catalyst doped with halogen compounds, characterised in that an aluminium hydroxide and/or aluminium oxide hydrate and/or silicic-acid gel is treated with excess solution of volatile ammonium salts, in order to absorb these salts, the aluminium hydroxide and/or aluminium oxide hydrate and/or silicic-acid gel thus treated is separated from the remaining solution of these salts, is dried at 60 to 200°C and calcined at 400 to 800°C with the expulsion of the volatile ammonium salts and with the formation of $Al_2O_3$ and/or $SiO_2$ and is then doped in a known manner with 0.1 to 20% by weight, based on the quantity of the $Al_2O_3$ and/or $SiO_2$, of halogen compounds of elements of the third to fifth main groups and second to eighth sub-groups of the periodic system (Mendeléeff).

9. Process according to Claim 8, characterised in that the volatile ammonium salt selected is that salt which consists of the cation of the ammonia used for the precipitation and the anion of the soluble aluminium salt used for the preparation of the aluminium hydroxide, this ammonium salt collecting on the freshly precipitated aluminium hydroxide by absorptive co-precipitation.

## Revendications

1. Procédé de production d'iso-alcènes par transformation catalytique de n-alcènes en mélange avec l'hydrogène et le cas échéant avec des gaz inertes à température élevée sur un catalyseur à base de $Al_2O_3$ et/ou de $SiO_2$ dopé avec des composés halogénés d'éléments des troisième à cinquième groupes principaux et des deuxième à huitième sous-groupes du système périodique (Mendelejew) et/ou avec des acides halogénhydriques, caractérisé en ce qu'on utilise une alumine et/ou une silice spécialement activée, l'activation spéciale résidant dans le fait qu'un hydroxyde d'aluminium et/ou un oxyde d'aluminium hydraté et/ou un gel de silice contenant des sels d'ammonium volatils avant le dopage sont calcinés en $Al_2O_3$ et/ou en $SiO_2$ avec élimination des sels d'ammonim volatils et ence qu'on opère dans un lit de catalyseur mobile et on remplace éventuellement le catalyseur, sans interrompre le procédé, en totalité ou en partie par du catalyseur neuf et/ou du catalyseur régénéré.

2. Procédé suivant la revendication 1, caractérisé en ce qu'on fait entrer le mélange en contact avec un catalyseur à base de $Al_2O_3$ et/ou de $SiO_2$ spécialement activé et dopé les composés halogénés mentionnés, déjà utilisé dans ce procédé, qui a été régénéré à une température de 400 à 800°C dans une atmosphère contenant de l'oxygène qui peut renfermer également des gaz inertes, avec addition d'hydrocarbures halogénés.

3. Procédé suivant la revendication 1, caractérisé en ce qu'il est mis en œuvre à une température de 400 à 600°C et pendant une durée de contact de 0,2 à 20 secondes.

4. Procédé suivant les revendications 1 à 3, caractérisé en ce que le catalyseur est disposé dans un lit mobile ou dans un lit tourbillonnaire.

5. Procédé suivant les revendications 1 à 4, caractérisé en ce que des quantités partielles du lit de catalyseur mobile sont déchargées, régénérées et recyclées dans le lit mobile de catalyseur.

6. Procédé suivant les revendications 1 à 5, caractérisé en ce que l'évacuation, la régénération et le recyclage sont effectués en continu.

7. Catalyseur à base d'un oxyde $Al_2O_3$ et $SiO_2$ qui est dopé avec 0,1 à 20% en poids, par rapport à la quantité de $Al_2O_3$ et/ou de $SiO_2$, de composés halogénés d'éléments du troisième à cinquième groupes principaux et du deuxième à huitième sous-groupes du système périodique (Mendelejew) et/ou d'acides halogénhydriques, caractérisé par une activation spéciale de $Al_2O_3$ et/ou de $SiO_2$, qui réside dans le fait qu'un hydroxyde d'aluminium et/ou un oxyde d'aluminium hydraté et/ou un gel de silice contenant des sels d'ammonium volatils avant le dopage sont calcinés en $Al_2O_3$ et/ou en $SiO_2$ avec élimination des sels d'ammonium volatils.

8. Procédé de production d'un catalyseur à base de $Al_2O_3$ et/ou de $SiO_2$ spécialement activé et dopé avec des composés halogénés, caractérisé en ce qu'on traite un hydroxyde d'aluminium et/ou un oxyde d'aluminium hydraté et/ou un gel de silice avec une solution en excès de sels d'ammonium volatils en vue de l'absorption de ces sels, on sépare l'hydroxyde d'aluminium et/ou l'oxyde d'aluminium hydraté et/ou le gel de silice ainsi traités de la solution résiduelle de ces sels, on sèche à 60—200°C et on calcine à 400—800°C avec élimination des sels d'ammonium volatils et avec formation de $Al_2O_3$ et/ou de $SiO_2$, puis on effectue le dopage d'une manière connue avec 0,1 à 20% en poids, par rapport à la quantité de $Al_2O_3$ et/ou de $SiO_2$, de composés halogénés d'éléments du troisième au cinquième groupes principaux et du deuxième au huitième sous-groupes du système périodique (Mendelejew).

9. Procédé suivant la revendication 8, caractérisé en ce qu'on choisit comme sel d'ammonium volatil le sel qui est formé à partir du cation de l'ammoniaque utilisée pour la précipitation et de l'anion du sel d'aluminium soluble utilisé pour la préparation de l'hydroxyde d'aluminium, ce sel d'ammonium s'additionnant alors par coprécipitation par absorption sur l'hydroxyde d'aluminium fraîchement précipité.

FIG.1

FIG. 2